# EUROPEAN PATENT SPECIFICATION

(11) **EP 2 249 815 B1**
(45) Date of publication and mention of the grant of the patent: **24.06.2020**
(21) Application number: 09704297.2
(22) Date of filing: 26.01.2009
(51) Int. Cl.: A61K 9/48, A61K 9/30

(54) **COMBINATIONS OF ORAL MEDICAMENTS BONDED BY A WRAPPING**
DURCH UMHÜLLUNG VERBUNDENE KOMBINATION VON ORALEN MEDIKAMENTEN
COMBINAISON DE MÉDICAMENTS ORAUX RÉUNIS PAR UNE ENVELOPPE

(30) Priority: 25.01.2008 FR 0850460; 25.01.2008 US 6651 P; 25.01.2008 US 6652 P; 25.01.2008 FR 0850461
(43) Date of publication of application: 17.11.2010
(73) Proprietor: Laboratoires Majorelle, 75008 Paris (FR)
(72) Inventor: LUGRIN, Anne-Emmanuelle, F-06600 Antibes (FR); GRISCELLI, Claude, F-75015 Paris (FR); EL GLAOUI, Mehdi, CH-1211 Geneve (CH); EL GLAOUI, Guillaume, F-75116 Paris (FR); HOFFELT, Jean, F-92250 La Garenne Colombes (FR)
(74) Representative: Icosa
(86) International application number: PCT/EP2009/050858
(87) International publication number: WO 2009/092819

(56) References cited:
- WO-A-01/08666
- WO-A-96/24375
- WO-A-97/25064
- CH-A- 552 510
- FR-A- 2 335 206
- FR-A- 2 524 311
- US-A- 5 897 910
- US-A1- 2004 170 567
- US-A1- 2005 053 648
- US-B1- 6 254 888
- YEBOAH-ANTWI K ET AL: "Impact of prepackaging antimalarial drugs on cost to patients and compliance with treatment." BULLETIN OF THE WORLD HEALTH ORGANIZATION 2001, vol. 79, no. 5, 2001, pages 394-399, XP009105031 ISSN: 0042-9686

## Description

The present invention relates to a novel oral pharmaceutical dosage form and to oral pharmaceutical combinations which are presented as said novel pharmaceutical dosage form and the purpose of which is to propose a novel therapeutic treatment method.

The large majority of proprietary medicines comprise a single active principle. The administration of several active principles to a patient therefore involves administering as many proprietary medicines.

From the patient's point of view, this gives rise to the problem of so-called compliance, and this question is becoming ever more problematic owing to the multiplication of coprescriptions, especially in the elderly.

The doctor himself is faced with the difficulty of coprescribing under the best conditions of effectiveness and safety, in so far as coprescriptions by the doctor are carried out somewhat empirically, especially in terms of population, posology and dosage.

The combination of a plurality of active principles in the same galenical form has been considered. gelatin capsules having two separate compartments or gelatin capsules nested one inside the others (FR2524311, US2005/053648) have been proposed, but these solutions have not been put into practice, doubtless because of expected difficulties in terms of handling, especially filling, handling of the separating caps, returns for filling and closure of the compartments.

Even more complex and specific forms have been conceived, as in WO01/08666, which describes assemblies provided with interconnection means which are to be fastened by welding. Also, in US20040170567 a dosage form containing an inactivating agent in combination with the medicament has been proposed. In this dosage form the two substances are separated by a permeable or semi-permeable partition, the partition becoming permeable upon activation.

Other techniques have been described, especially in FR2335206, which describes a pharmaceutical dosage form in which the medicaments are deposited (by spraying or electrostatically) on continuous edible strips of paper and/or of polymer material. The galenical form comprises a plurality of layers of edible sheets, which can carry one or more active principles, the layers or sheets are so arranged that they do not exhibit active principle on the surface that is to constitute the outer surface of the galenical form, and they are sealed so as to trap the active principle(s) inside.

US5,897,910 aims to make the production of film-coated tablets simpler and more economical. It describes a method for the production of film-coated tablets in which the formation of the tablet and the film coating thereof with the aid of a sheet of material are carried out simultaneously.

In respect of tablets, multilayers, for example in WO97/25064 and WO96/24375, especially bilayers, have also been proposed. However, multilayer technology is also difficult to implement if the dosage of each medicament is to be respected very precisely, given the problems of stability and expiry of medicaments.

It is an object of the invention, therefore, to remedy those disadvantages by proposing a novel oral pharmaceutical dosage form which brings together two or more medicaments and which is simple to manufacture.

Another object of the invention is to remedy the above-described disadvantages by proposing a combination of medicaments in a novel pharmaceutical dosage form which is simple to manufacture.

Another object of the invention is to provide such a pharmaceutical dosage form which is likely to simplify the procedures of registration as a medicament.

Another object of the invention is to provide such a form which is especially designed to correspond to very precise dosages, posologies and patients and simplify the work of prescribing for the doctor.

Yet another object of the invention is to propose a solution which allows the dosage of each of the combined medicaments to be made certain.

Yet another object of the invention is to propose a solution which makes it possible not to modify the bioavailability of the combined medicaments.

Yet another object of the invention is to propose a versatile solution which allows different galenical forms or forms having different weights or volumes to be combined.

Those objects are achieved by the invention, which relates to an oral pharmaceutical dosage form containing at least two medicaments, in which form the medicaments on the one hand are brought together in a leakproof water soluble wrapping or envelop and on the other hand are separated so that the active principles of the combined medicaments cannot come into contact with one another.

Within the scope of the invention, water-soluble means that the wrapping is dissolvable and dissolved within a cavity of the digestive system, e.g. where absorption of the combined medicaments is desired, it being possible for the cavity to be the mouth, the stomach or the intestine. The cavity may be the stomach, it being possible for the medicaments to be absorbed in the stomach or in the intestine, including at least one in the stomach and at least one in the intestine. In a particular embodiment, the wrapping dissolves rapidly and particularly instantaneously in water or in saliva. The wrapping is said to be water-soluble in in vivo.

The wrapping is called leakproof in order to isolate the medicaments from the ambient medium. Especially, the wrapping isolates the medicaments from ambient moisture. Isolation can be improved in the manner known *per se* by the packaging of the pharmaceutical dosage form, for example in a blister or any other known means. The wrapping protects the pharmaceutical dosage form until it is ingested by the patient.

The medicaments are preferably separated by a sheet or wall which extends inside the wrapping. The wrapping and the sheet or wall, where the last is present, are, of course, made of pharmaceutical (pharmaceutically acceptable) material. The wrapping and the sheet or wall are in contact in order to provide continuity of separation and protection.

Within the scope of the invention, a medicament is understood as being a galenical or galenic formulation (or dosage form) of an active principle, that is so say the mixture of the active principle and one or more excipients, adjuvants, carriers, substrates, in a solid oral galenical form. The medicaments or galenical forms are preconstituted. A solid oral galenical form is understood as being a solid oral form of administration, that is to say a form which is conventionally suitable for administration such as by the oral route. This includes especially the forms of tablet, coated tablet, gelatin capsule or soft capsule. This excludes a powdered or liquid composition which would not be included in a solid oral galenical form. After dissolution of the wrapping, the various galenical formulations or medicaments are released into a body cavity in their original form, as if each of the medicaments had been administered separately from the other(s).

According to a feature of the invention, the dosages of the medicaments so combined are coordinated. This means that the dosages of the associated medicaments are optimised according to an optimum therapeutic effectiveness in a single administration.

The oral pharmaceutical dosage form of the invention can comprise especially, without being limited, two, three or four medicaments.

The pharmaceutical dosage forms according to the invention can have one, a plurality or all of the following features:
- the medicaments are commercial galenical forms;
- the medicaments are galenical forms similar to commercial galenical forms in the sense that they have the same qualitative and quantitative composition but a different geometric form;
- combination of at least one commercial galenical form and at least one similar
- galenical form;
- the wrapping and optionally the separating sheet or wall is made of material that is water-soluble under the pH conditions of the predetermined absorption site; this can be the mouth, the stomach or the intestine; the separating sheet or wall, unlike the wrapping, need not necessarily be made of water-soluble material; it must, however, be made of pharmaceutical material that is compatible with the material of the wrapping; it is therefore more convenient to use the same material;
- the water-soluble material is water-soluble at the pH of the mouth (of saliva), that is to say a pH of the order of from 7 to 8;
- the water-soluble material is water-soluble at the pH of the stomach (very acidic pH, generally about 1);
- the water-soluble material is water-soluble at the pH of the intestine, that is to say an alkaline pH (about 8 to 9);
- the combined medicaments are to be absorbed in the same cavity of the digestive system, or in different cavities;
- the pharmaceutical dosage form is limited to a total weight of medicament of less than or equal to 1500 mg, preferably less than or equal to 1000 mg, especially less than or equal to 800, 700 or 600 mg, in particular less than or equal to 500 or 400 mg;
- the pharmaceutical dosage form has a total volume which is compatible with easy swallowing for forms other than those which disintegrate in the mouth;
- the galenical forms of the combined medicaments are of the same nature; for example, selected from tablets, coated tablets, gelatin capsules or soft capsules;
- the galenical forms of the combined medicaments are of different natures, for example for the combination of two medicaments: tablet and coated tablet, tablet and gelatin capsule, tablet and soft capsule, and gelatin capsule, coated tablet and soft capsule, soft capsule and gelatin capsule;
- in the case especially where at least one of the medicaments is a capsule or a gelatin capsule, the separating sheet or wall can be omitted, physical separation in this case being ensured by the wall of the gelatin capsule or capsule;
- the weight of the combined medicaments is substantially identical or is different;
- the volume of the combined medicaments is substantially identical or is different.

According to a first embodiment, the wrapping is of the soft capsule type and is obtained from two half-capsules of gelatin or an analogous material, which are sealed together around the combined medicaments, preferably with separation by a separating sheet or wall (or more depending on the number of medicaments), the latter preferably being made from the same material as the capsule, for example of gelatin or the like. The production process for a two-in-one form can comprise the use of two conventional honeycomb rollers. The counter-rotating rollers are juxtaposed in a manner known per se. The rollers are supplied with three strips of gelatin or the like (two for the wrapping and a central one for the separation) and with the two medicaments. Sealing of the strips of gelatin or the like is carried out by the pressure exerted between the two rollers.

According to a second disclosure, the wrapping is a matrix of rice paper or analogous material, especially a matrix formed by one part, for example an oblong part, having at least two compartments, each of which receives a medicament, and one part forming a cover. Closing can be effected by simple nesting or by bonding, for example wet bonding according to the method conventional for bonding that material.

According to a third disclosure, the wrapping is an inclusion matrix, the medicaments being included in that matrix. The method can consist in bringing the medicaments together in a mould into which a liquid binder is poured in an appropriate manner, optionally in two phases, which binder, after curing, will form a matrix that is water-soluble under the appropriate pH conditions. The matrix can especially be made of gelatin or the like or alternatively of a polymeric material such as a polyethylene glycol, for example PEG 6000.

According to a fourth embodiment, which is preferred, the wrapping is formed by two sheets of water-soluble material which are applied so as to surround the combined medicaments. They are then sealed to one another.

In this embodiment, it is preferred to use one or more sheet(s), preferably of the same material, to separate the medicaments from one another inside the wrapping. The sheet or sheets can advantageously be sealed to the edges of the sheets of the wrapping at the same time as the edges are sealed to one another.

Accordingly, in a preferred form of this embodiment, the pharmaceutical dosage form comprises at least two medicaments, for example two, a wrapping produced from two sheets of pharmaceutical material and at least one (if there are two medicaments, or one or two if there are three medicaments, etc.) separating sheet made of pharmaceutical material, the three sheets being sealed together over the entire periphery of the pharmaceutical dosage form.

The sheets of pharmaceutical material for forming the wrapping and optionally the separating sheets or walls can have one, a plurality or all of the following features:
- they are of the same nature for the separating sheet and the sheets of the wrapping;
- they are sealed at their edges;
- they are water-soluble at the pH of the predetermined absorption site;
- they are water-soluble at the pH of the mouth (saliva), that is to say a pH of the order of from 7 to 8;
- they are water-soluble at the pH of the stomach (very acidic pH, generally about 1);
- they are water-soluble at the pH of the intestine, that is to say an alkaline pH (pH approximately 8 to 9);
- they are made of pharmaceutical material for oral ingestion;
- they are formed of or comprise one of the following materials: biomaterials, especially biomaterials obtained from or based on algae, cellulose polymers or copolymers, polyvinyl alcohol, lactic and/or glycolic acid derivatives, especially PLGA and PLA, polycaprolactone (PCL);
- the material can undergo slight shrinkage, after sealing, in order to follow the shape of the encased tablets in the best possible manner;
- thickness of the material: especially from 30 to 300 µm.

The edges of the sheets are to be sealed together over the entire periphery, and the sealed edges are preferably turned down as close to the form as possible so as not to create a relief which would be detrimental to pleasant ingestion (mouth) or to easy swallowing (stomach, intestine). If necessary, before the edges are turned down, they can be made smaller by cutting.

The method of sealing the edges is adapted to the material of the sheets.

According to a fifth disclosure, which is suitable for tablets, the wrapping is a film coating present over the entire outer surface of the tablets brought together by bonding. The tablets have a planar surface, preferably complementary to that of the associated tablet. If three tablets are combined, the middle tablet has two planar surfaces.

In that case, the pharmaceutical dosage form comprises at least two tablets brought together by a separating film coating or bonding and an encasing film coating, the two film coatings advantageously being continuous material. The two film coatings can be of the same material or of materials that are different but are advantageously compatible in order to ensure continuity of material. Conventional film coating compositions and methods can be used to produce them.

According to a first form, the tablets are wholly film-coated and the film-coated tablets are bonded to one another by a surface that is sufficient to ensure a unitary configuration until they are dissolved in the body cavity.

According to a second form, the tablets are first bonded together and then the whole is film-coated.

The method preferably comprises the separate film coating of the two tablets, for example by spraying of a film-forming solution. That solution can comprise a copolymer of polyvinyl alcohol and polyethylene glycol. Advantageously, the wrapping and especially the one obtained from said copolymer is rapidly or instantaneously solubilised in aqueous environment at the time the dosage form is ingested. Preferably, the film-forming solution does not modify the solubility parameters of the original tablets. Each tablet can advantageously be film-coated with the aid of its own coloured solution so that the presence of the different tablets is visible to the naked eye.

Bonding can then be carried out with the aid of a suitable bonding solution. That solution can be of the same nature as the film-forming solution, the dilution or viscosity optionally being different. The solution can be of a different nature and comprise other suitable monomers or polymers. The bonding solution can be an aqueous solution or a solution obtained by using a gas maintained in the supercritical liquid state, such as, for example, carbon dioxide. Bonding itself can be obtained, for example, by simple contact or by fusion by compression, or by another known method.

Bonding can also be effected with the aid of a pulverulent substance placed between the tablets, followed by fusion by compression.

The tablets or coated tablets combined in the same form can have one, a plurality or all of the following features:
- if they are tablets and/or coated tablets, they each have a face that is complementary in terms of form with a face of the other, the two faces being intended to be opposite one another in the final pharmaceutical dosage form; those two faces are preferably planar or substantially planar;
- the tablets and/or coated tablets each have a planar or substantially planar face and the two faces have the same geometry;
- the two complementary faces have a round or ovoid/oval geometry;
- the final pharmaceutical dosage form substantially forms a sphere or an elongated sphere (oblong form);
- the tablets/coated tablets have substantially identical weights;
- the tablets/coated tablets have different weights.

According to a feature of the invention, the geometric form of the medicament is adapted to the needs of the invention. If an existing medicament (reference proprietary medicine) is used, the adaptation is made without changing the qualitative and quantitative composition of the original medicament ("similar galenical form"). Accordingly especially, starting from the active principle and the excipient(s) of the reference proprietary medicine, tablets having the desired form are produced. The two tablets to be combined are produced so that they have the optimum complementary geometric forms described in this specification.

The invention relates also to oral pharmaceutical combinations which are presented in the pharmaceutical dosage form according to the invention and the purpose of which is to propose a therapeutic treatment method permitting the joint administration of medicaments (especially two, three or four medicaments), in which an oral pharmaceutical dosage form according to the invention is administered to a patient who needs it. The various pharmaceutical dosage forms mentioned above can be the subject of that method.

According to a feature of the invention, the doses of the medicaments so combined are coordinated. This means that the dosages of the various combined medicaments correspond to the medical prescription which defines the respective doses of each of the medicaments for optimum therapeutic effectiveness in a single administration.

More particularly, the invention relates to an oral pharmaceutical dosage form containing at least two medicaments, in which form the medicaments on the one hand are brought together in the water-soluble wrapping and on the other hand are separated so that they do not come into contact with one another, at least one of the medicaments being selected from the following therapeutic classes: nonsteroidal anti-inflammatory drug (NSAID), proton pump inhibitor (PPI), betablocker, statin, conversion enzyme inhibitor (CEI), biguanide, myorelaxant, calcium inhibitor, corticoid, antidepressant, benzodiazepine, non-atropine-like intestinal transit retarder, intestinal antibacterial, and from the following therapeutic molecules: spironolactone, aldactone, propranolol, clarithromycin, amoxycillin, low-dose acetylsalicylic acid (aspirin), carvedilol, potassium, clopidogrel.

The invention relates especially to combinations of two, three or four medicaments.

The medicaments can be chosen to constitute the following preferred combinations:
- a non-steroidal anti-inflammatory drug (NSAID) combined with a proton pump inhibitor (PPI);
- a beta-blocker combined with a statin;
- a conversion enzyme inhibitor (CEI) combined with a statin;
- a statin combined with a biguanide;
- an NSAID combined with a myorelaxant;
- a calcium inhibitor combined with a CEI;
- spironolactone combined with propranolol;
- a PPI combined with clarithromycin and amoxycillin;
- low-dose acetylsalicylic acid ("infant-type" dose, which has an effect of fluidising the blood in adult patients and is commonly used in cardiology to treat problems of hypertension; dose from 75 mg, for example from 75 to 160 mg dose) combined with clopidogrel;
- low-dose acetylsalicylic acid combined with a CEI, a statin and a beta-blocker;
- a corticoid (doses varying from 20 to 100 mg especially) combined with potassium;
- an antidepressant combined with a benzodiazepine (anxiolytic);
- a non-atropine-like intestinal transit retarder combined with an intestinal antibacterial.

The invention relates to an oral pharmaceutical dosage form containing a medicament comprising an NSAID and a medicament comprising a PPI.

The indication is the anti-inflammatory treatment of patients at risk of gastroduodenal lesions.

The invention relates also to the use of a medicament comprising an NSAID and of a medicament comprising a PPI in the preparation of a two-in-one oral pharmaceutical dosage form according to the invention as an anti-inflammatory for patients at risk of gastroduodenal lesions.

As NSAID there may be mentioned especially, without being limited: naproxen, ketoprofen, diclofenac, ibuprofen.

As PPI there may be mentioned especially, without being limited: omeprazole, esomeprazole and lansoprazole.

The invention relates also to an oral pharmaceutical dosage form containing a medicament comprising a beta-blocker and a medicament comprising a statin.

The indication is hypercholesterolaemia in patients with arterial hypertension.

The invention relates also to the use of a medicament comprising a beta-blocker and of a medicament comprising a statin in the preparation of a two-in-one oral pharmaceutical dosage form according to the invention for the treatment of hypercholesterolaemia in patients with arterial hypertension.

As beta-blocker there may be mentioned especially, without being limited: bisoprolol, atenolol, acebutolol.

As statin there may be mentioned especially, without being limited: pravastatin, simvastatin.

The invention relates also to an oral pharmaceutical dosage form containing a medicament comprising a CEI and a medicament comprising a statin.

The indication is the prevention of cardiovascular disorders in at-risk subjects, especially in arterial hypertension associated with hypercholesterolaemia.

The invention relates also to the use of a medicament comprising a CEI and of a medicament comprising a statin in the preparation of a two-in-one oral pharmaceutical dosage form according to the invention for the prevention of cardiovascular disorders in at-risk subjects, especially in arterial hypertension associated with hypercholesterolaemia.

As statin there may be mentioned especially, without being limited: pravastatin, simvastatin.

As CEI there may be mentioned especially, without being limited: ramipril, captopril, enalapril and fosinopril.

The invention relates also to an oral pharmaceutical dosage form containing a medicament comprising a biguanide and a medicament comprising a statin.

The indication is the prevention of cardiovascular risks in patients with type 2 diabetes.

The invention relates also to the use of a medicament comprising a biguanide and of a medicament comprising a statin in the preparation of a two-in-one oral pharmaceutical dosage form according to the invention for the prevention of cardiovascular risks in patients with type 2 diabetes.

As statin there may be mentioned especially, without being limited: pravastatin, simvastatin.

As biguanide there may be mentioned especially, without being limited: metformin, gliclazide, carbutamide and glibenclamide.

The invention relates also to an oral pharmaceutical dosage form containing a medicament comprising an NSAID and a medicament comprising a myorelaxant.

The indication is the treatment of common acute lumbar pain.

The invention relates also to the use of a medicament comprising an NSAID and of a medicament comprising a myorelaxant in the preparation of a two-in-one or three-in-one oral pharmaceutical dosage form according to the invention for the treatment of common acute lumbar pain.

As myorelaxant there may be mentioned especially, without being limited: thiocolchicoside.

As NSAID there may be mentioned especially, without being limited: naproxen, ketoprofen, diclofenac, ibuprofen.

The invention relates also to an oral pharmaceutical dosage form containing a medicament comprising a calcium inhibitor and a medicament comprising a CEI.

The indication is the treatment of arterial hypertension resistant to monotherapy.

The invention relates also to the use of a medicament comprising a calcium inhibitor and of a medicament comprising a CEI in the preparation of a two-in-one oral pharmaceutical dosage form according to the invention for the treatment of hypertension resistant to monotherapy,

As CEI there may be mentioned especially, without being limited: ramipril, captopril, enalapril and fosinopril.

As calcium inhibitor there may be mentioned especially, without being limited: nifedipine, felodipine and nicardipine.

The invention relates also to an oral pharmaceutical dosage form containing a medicament comprising spironolactone and a medicament comprising propranolol.

The indication is the treatment of cirrhosis in patients with pulmonary arterial hypertension.

The invention relates also to the use of a medicament comprising spironolactone and of a medicament comprising propranolol in the preparation of a two-in-one oral pharmaceutical dosage form according to the invention for the treatment of cirrhosis in patients with pulmonary arterial hypertension.

The invention relates also to an oral pharmaceutical dosage form containing a medicament comprising a PPI, a medicament comprising clarithromycin and a medicament comprising amoxycillin.

The indication is the eradication treatment of the bacterium *Helicobacter pylori* in the case of gastroduodenal ulcer disease in adults; proof of lesion and infection is usually provided by endoscopy.

The invention relates also to the use of a medicament comprising a PPI, of a medicament comprising clarithromycin and of a medicament comprising amoxycillin in the preparation of a three-in-one oral pharmaceutical dosage form according to the invention for the eradication of the bacterium *Helicobacter pylori* in the case of gastroduodenal ulcer disease in adults.

As PPI there may be mentioned especially, without being limited: omeprazole, esomeprazole and lansoprazole.

The invention relates also to an oral pharmaceutical dosage form containing a medicament comprising low-dose acetylsalicylic acid, a medicament comprising a CEI, a medicament comprising a statin and a medicament comprising a betablocker.

The indication is post-infarct treatment (with or without cardiac insufficiency).

The invention relates also to the use of a medicament comprising low-dose acetylsalicylic acid, of a medicament comprising a CEI, of a medicament comprising a statin and of a medicament comprising a beta-blocker in the preparation of a four-in-one oral pharmaceutical dosage form according to the invention for post-infarct treatment.

As beta-blocker there may be mentioned especially, without being limited: bisoprolol, atenolol, acebutolol, carvedilol.

As statin there may be mentioned especially, without being limited: pravastatin, simvastatin.

As CEI there may be mentioned especially, without being limited: ramipril, captopril, enalapril and fosinopril.

The invention relates also to an oral pharmaceutical dosage form containing a medicament comprising low-dose acetylsalicylic acid and a medicament comprising clopidogrel.

The indication is the prevention of the risk of thromboembolism in hypertensive patients and in patients who have undergone angioplasty.

The invention relates also to the use of a medicament comprising low-dose acetylsalicyclic acid and of a medicament comprising clopidogrel in the preparation of a two-in-one oral pharmaceutical dosage form according to the invention for the prevention of the risk of thromboembolism in hypertensive patients and in patients who have benefited from an angioplasty.

The invention relates also to an oral pharmaceutical dosage form containing a medicament comprising a corticoid and a medicament comprising potassium.

The indication is the prevention of hypokaliaemia in patients who are to be treated 5 by corticotherapy.

The invention relates also to the use of a medicament comprising a corticoid and of a medicament comprising potassium in the preparation of a two-in-one oral pharmaceutical dosage form according to the invention for the prevention of hypokaliaemia in patients who are to be treated by corticotherapy.

The corticoid can be used in doses varying from 20 to 100 mg.

As corticoid there may be mentioned especially, without being limited: prednisolone, prednisone.

Potassium is generally in the form of a potassium salt. As potassium salt there may be mentioned especially potassium chloride.

The invention relates also to an oral pharmaceutical dosage form containing a medicament comprising an antidepressant and a medicament comprising a benzodiazepine.

The indication is the treatment of depression.

The invention relates also to the use of a medicament comprising an antidepressant and of a medicament comprising a benzodiazepine in the preparation of a three-in-one oral pharmaceutical dosage form according to the invention for the treatment of depression.

The antidepressant can be especially a tricyclic antidepressant, for example clomipramine, or a selective serotonin reuptake inhibitor, for example fluoxetine or venlafaxine.

The benzodiazepine (anxiolytic) can be, without being limited: bromazepan, alprazolam.

The invention relates also to an oral pharmaceutical dosage form containing a medicament comprising a non-atropine-like intestinal transit retarder and a medicament comprising an intestinal antibacterial.

The indication is the reestablishment of intestinal transit and treatment of bacterial diarrhoea.

The invention relates also to the use of a medicament comprising a non-atropine like intestinal transit retarder and of a medicament comprising an intestinal antibacterial in the preparation of a two-in-one oral pharmaceutical dosage form according to the invention for re-establishing intestinal transit and the treatment of bacterial diarrhoea.

As non-atropine-like intestinal transit retarder there may be mentioned for example loperamide.

As intestinal antibacterial there may be mentioned, for example, nifuroxazide.

For each of the precise combinations which have just been described, the medicaments are preferably brought together in a water-soluble wrapping and are separated so that their active principles cannot come into contact with one another.

The medicaments mentioned within the context of those various combinations can be in the various galenical forms mentioned above.

The medicaments according to the invention are or can be the subject of commercial forms which can be used as such for implementing the invention.

Among the commercial forms or reference specialities which can be used there may be mentioned, for example:
- Elisor® (pravastatin), Zocor® (simvastatin) for the statins (class C10A1)
- Mopral® (omeprazole) for the proton pump inhibitor (class A2B2);
- Naprosyne® (naproxen), Nurofen® (ibuprofen), Ketum® (ketoprofen) and Voltaren® (diclofenac) for the non-steroidal antiinflammatory drugs (M1A1);
- Detensiel® or Soprol® (bisoprolol), Tenormine® (atnolol), Sectral® (acebutolol) for the beta-blockers (class C7A); Kredex® (carvedilol);
- Triatec® (ramipril), Lopril® (captopril), Renitec® (enalapril), Fositec® (fosinopril) for the conversion enzyme inhibitors (CEI, class C9A);
- Glucophage® (metformin) and Diamicron® (gliclazide) for the biguanides (class A1082);
- Solupred®, Cortancyl®, Medrol® (corticoids);
- Kaleorid®, Difuca® (potassium salt);
- Zeclar® or Naxy® (clarithromycin), Clamoxyl® or Agram® (amoxycillin);
- Aldactone® 50 (spironolactone), Avlocardyl® 40 (propranolol);
- Adalat® LD (nifedipine), Flodil® (felodipine), Loxen® (nicardipine);
- Imossel® and Imodium® (loperamide), Ercefuryl® (nifuroxozide);
- Plavix® (clopidrogel);
- Anafranil® (clomipramine), Prozac® (fluoxetine), Effexor® (venlafaxine);
- Lexomil® (bromazepan), Xanax® (alprazolam)®;
- or their generic proprietary medicine

However, according to one feature of the invention, the geometric form of the medicaments can be adapted to the needs of the invention. If an existing medicament (reference generic proprietary medicine) is used, the adaptation is made without changing the qualitative and quantitative composition of the original medicament ("similar galenical form"). Accordingly especially, starting from the active principle and the excipient(s) of the reference proprietary medicine, tablets having the desired form are produced. The two tablets to be combined are produced so that they have the optimum complementary geometric forms described in this specification.

The invention relates also to a therapeutic treatment method which permits the joint administration of medicaments (especially two, three or four medicaments), in which an oral pharmaceutical dosage form according to the invention is administered to a patient who needs it, for the indicated therapeutic indication. The various pharmaceutical dosage forms mentioned above and their therapeutic indications as presented in this specification can be the subject of this method.

According to one feature of the invention, the medicaments have coordinated therapeutic dosage. This means that the dosages of the associated medicaments are optimised according to an optimum therapeutic effectiveness in a single administration.

A first embodiment accordingly consists in administering an oral pharmaceutical dosage form containing a medicament comprising an NSAID and a medicament comprising a PPI. The indication is anti-inflammatory treatment in patients at risk of gastroduodenal lesions. Medicaments and combinations which can be used have been described *supra.*

A second embodiment consists in administering an oral pharmaceutical dosage form containing a medicament comprising a beta-blocker and a medicament comprising a statin. The indication is hypercholesterolaemia in patients with arterial hypertension. Medicaments and combinations which can be used have been described *supra.*

A third embodiment consists in administering an oral pharmaceutical dosage form containing a medicament comprising a CEI and a medicament comprising a statin. The indication is the prevention of cardiovascular disorders in at-risk subjects, especially in the case of arterial hypertension associated with hypercholesterolaemia. Medicaments and combinations which can be used have been described *supra.*

A fourth embodiment consists in administering an oral pharmaceutical dosage form containing a medicament comprising a biguanide and a medicament comprising a statin. The indication is the prevention of cardiovascular risks in patients with type 2 diabetes. Medicaments and combinations which can be used have been described *supra.*

A fifth embodiment consists in administering an oral pharmaceutical dosage form containing a medicament comprising an NSAID and a medicament comprising a myorelaxant. The indication is the treatment of common acute lumbar pain. Medicaments and combinations which can be used have been described *supra.*

A sixth embodiment consists in administering an oral pharmaceutical dosage form containing a medicament comprising a calcium inhibitor and a medicament comprising a CEI. The indication is the treatment of arterial hypertension resistant to monotherapy. Medicaments and combinations which can be used have been described *supra.*

A seventh embodiment consists in administering an oral pharmaceutical dosage form containing a medicament comprising spironolactone and a medicament comprising propranolol. The indication is the treatment of cirrhosis in patients with pulmonary arterial hypertension. Medicaments and combinations which can be used have been described *supra.*

An eighth embodiment consists in administering an oral pharmaceutical dosage form containing a medicament comprising a PPI, a medicament comprising clarithromycin and a medicament comprising amoxycillin. The indication is the eradication treatment of the bacterium *Helicobacter pylori* in the case of gastroduodenal ulcer disease in adults; proof of lesion and infection is usually provided by endoscopy. Medicaments and combinations which can be used have been described *supra.*

A ninth embodiment consists in administering an oral pharmaceutical dosage form containing a medicament comprising low-dose acetylsalicylic acid, a medicament comprising a CEI, a medicament comprising a statin and a medicament comprising a beta-blocker. The indication is post-infarct treatment (with or without cardiac insufficiency). Medicaments and combinations which can be used have been described *supra.*

A tenth embodiment consists in administering an oral pharmaceutical dosage form containing a medicament comprising low-dose acetylsalicylic acid and a medicament comprising clopidogrel. The indication is the prevention of the risk of thromboembolism in hypertensive patients and in patients who have undergone angioplasty. Medicaments and combinations which can be used have been described *supra.*

An eleventh embodiment consists in administering an oral pharmaceutical dosage form containing a medicament comprising a corticoid and a medicament comprising potassium. The indication is the prevention of hypokaliaemia in patients requiring treatment by corticotherapy. Medicaments and combinations which can be used have been described *supra.*

A twelfth embodiment consists in administering an oral pharmaceutical dosage form containing a medicament comprising an antidepressant and a medicament comprising a benzodiazepine. The indication is the treatment of depression. Medicaments and combinations which can be used have been described *supra.*

A thirteenth embodiment consists in administering an oral pharmaceutical dosage form containing a medicament comprising a non-atropine-like intestinal transit retarder and a medicament comprising an intestinal antibacterial. The indication is the reestablishment of intestinal transit and the treatment of bacterial diarrhoea. Medicaments and combinations which can be used have been described supra.

The present invention relates also to a process for producing oral pharmaceutical dosage forms according to the invention. The process comprises a step of bringing the two or more medicaments together in the leakproof water-soluble wrapping and optionally a step of separating the medicaments by interposing a sheet or wall; that step preceding or being simultaneous with the step of bringing together.

Details relating to the production of each of the embodiments of the pharmaceutical dosage forms according to the invention are given in the remainder of the description.

A feature of the process is the bringing together of two or more preconstituted, especially existing or commercial, medicaments or galenical forms.

According to another feature, the format of at least one of the medicaments is modified in order to adapt it to the format of the other in order to produce a pharmaceutical dosage form that is as compact as possible and easy to ingest. Especially, the formats of the two or more medicaments are adapted.

For example, the tablets are chosen or formatted to have planar surfaces opposite one another. They are also chosen or formatted to have planar surfaces whose sizes and forms are similar, or even better, identical.

The invention will now be described with the aid of embodiments given by way of nonlimiting example and with reference to the accompanying drawings.

Figures 1, 2 and 3 show three cross-sectional representations, in diagrammatic form, of pharmaceutical dosage forms according to the invention, using sheets of pharmaceutical material and prior to sealing of the edges.

Figures 4 and 5 are cross-sectional representations, in diagrammatic form, of a disclosure using a matrix of rice paper.

Figure 6 shows, in diagrammatic form, a disclosure by inclusion in a water-soluble matrix.

Figure 7 shows, in diagrammatic form, a disclosure of two tablets associated using a coating.

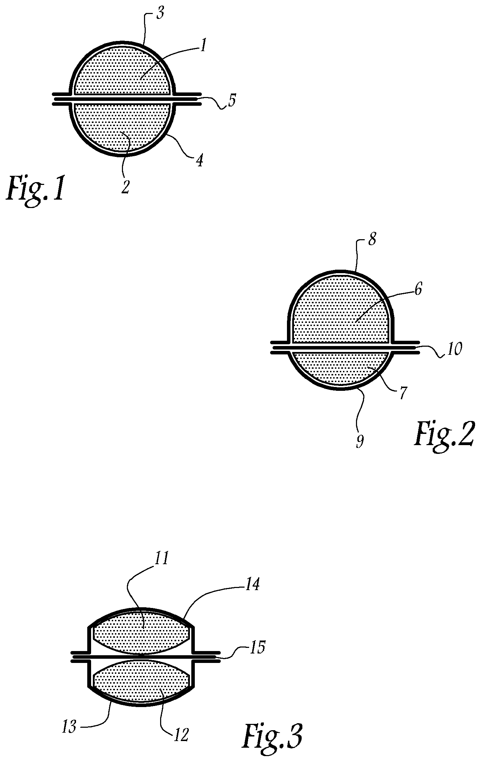

Figure 1 shows a pharmaceutical dosage form comprising two tablets 1 and 2 having the same hemispherical form and having the same volume, for example 200 mg each.

Combination to give a substantially spherical single pharmaceutical dosage form is obtained by using 3 sheets of pharmaceutical film of cellulose copolymer, denoted by the reference numerals 3, 4 and 5, reference numeral 5 denoting the separating sheet between the planar faces of the tablets 1 and 2. For the three embodiments shown, the edges of the three sheets are to be sealed together over the entire periphery, and the sealed edges are turned down as close to the form as possible so as not to create a relief which is detrimental to easy swallowing. If necessary, the edges can be made smaller by cutting before they are turned down.

By way of variation, two commercially available tablets are used without modifying their form.

Figure 2 shows a pharmaceutical dosage form comprising two tablets 6 and 7 having different volumes, for example 400 mg and 200 mg, respectively. Combination to give a substantially spherical single pharmaceutical dosage form is obtained using 3 polymer sheets, for example of polyvinyl alcohol, denoted by the reference numerals 8, 9 and 10, reference numeral 10 denoting the separating sheet between the planar faces of the tablets 6 and 7.

By way of variation, two commercially available tablets are used without modifying their form.

Figure 3 shows a pharmaceutical dosage form comprising two tablets 11 and 12 having the same form and having the same volume, for example 300 mg each. Combination to give a single pharmaceutical dosage form is obtained using 3 polycaprolactone sheets denoted by the reference numerals 13, 14 and 15, reference numeral 15 denoting the separating sheet between the planar faces of the tablets 11 and 12.

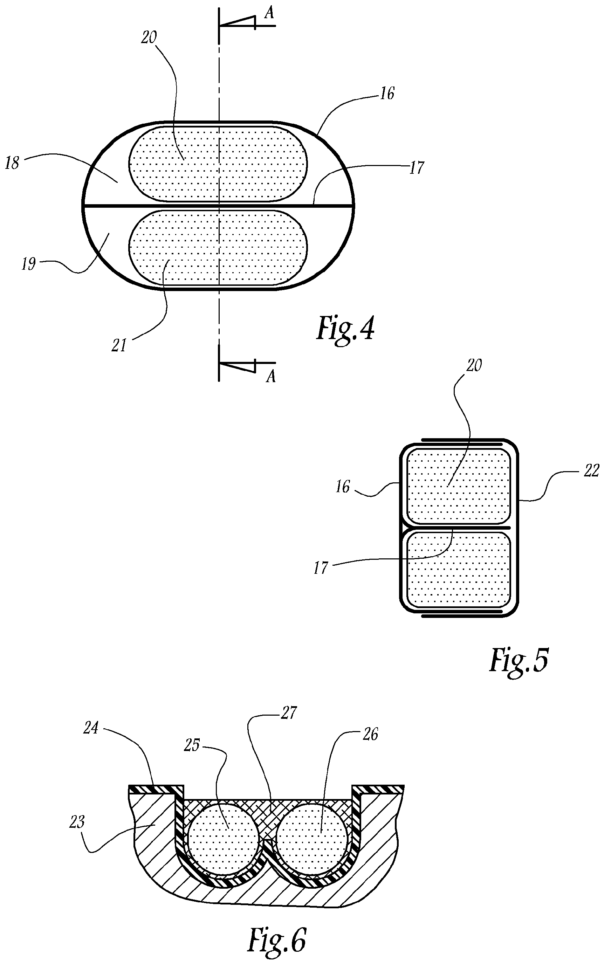

Figure 4 shows the body of a matrix of rice paper, which is not part of the present invention, comprising an outer wrapping 16 and a separating wall 17, the whole delimiting two compartments 18 and 19. Each of the compartments of the body is shown containing a galenical form 20, 21. The matrix comprises a flat base (not shown) and a cover (not shown). Figure 5 shows a cross-sectional view according to AA of Figure 4, in which there is this time shown not only the body of the matrix but also its cover 22. The cover 22 is fitted onto the body of the matrix. It will be noted that the matrix body, as well as the cover, can be made in the conventional manner, by moulding a rice paper solution and curing.

Figure 6 shows a steel mould 23, which is not part of the present invention, a blister cell 24 of PVC or PVC-PVDC or of any other alimentary plastics material suitable for forming a blister cell. This cell may or may not be coated with one or more protective films. It can simply be thermoformed in the mould 23. The two medicaments 25 and 26 are placed in the cell, and then the binder, which will form the inclusion matrix 27, is poured in. The matrix is then cured by cooling. If the matrix is made of gelatin, drying is also provided. If the matrix is made of PEG, for example PEG 6000, curing is simply carried out by cooling. The cell is then closed by a sealing sheet, conventionally by thermosealing of an aluminium complex (not shown).

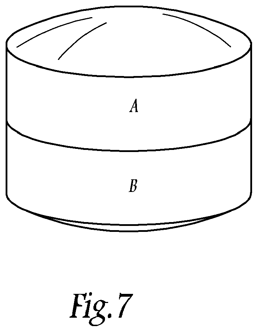

Figure 7 shows a pharmaceutical dosage form, which is not part of the present invention, obtained by a process of film coating and bonding. Each of the tablets A and B is film-coated individually in a revolving drum by spraying of a film-forming solution obtained, for example, from a copolymer of polyvinyl alcohol and polyethylene glycol. The spraying solutions have a different colour for each of the tablets.

Bonding is then carried out by deposition of a solution obtained with the film-forming substance. The concentration of the bonding solution is adjusted, if necessary, in order to have a suitable viscosity. Bonding is carried out by simple contact or by compression fusion.

The present specification focuses substantially on the combination of two medicaments. The person skilled in the art will easily be able to adapt the pharmaceutical dosage form to the presence of more than two medicaments, especially three or four medicaments, by incorporating as many separating sheets or walls as are necessary. It will be possible for the person skilled in the art to envisage pharmaceutical dosage forms other than those which have just been described. For example, he will be able to employ gelatin capsules, and in particular multi-compartment gelatin capsules, without the disadvantages associated with the difficulties of filling and handling that are encountered in the prior art, because a medicament within the scope of the invention, that is to say a solid oral galenical form, is placed in each compartment. It will then be possible, for example, to assemble the parts of gelatin capsules on a horizontal plane and under industrially acceptable conditions. Such a gelatin capsule therefore comprises at least two compartments each containing a medicament according to the invention, which are separated from one another by a separating wall.

## Claims

1. Oral pharmaceutical dosage form containing at least two medicaments, in which form the medicaments are under the form of separately preconstituted tablets which on the one hand are brought together in a leakproof *in vivo* water-soluble wrapping and on the other hand are separated so that the active principles in the combined medicaments cannot come into contact with one another, and wherein the wrapping is made from two sheets of pharmaceutical material, and at least one separating sheet made of pharmaceutical material.

2. Pharmaceutical dosage form according to claim **1**, in which the medicaments are commercial tablets and/or tablets having the same qualitative and quantitative composition as commercial tablets but a different geometric form.

3. Pharmaceutical dosage form according to claim **1** or **2**, in which the medicaments are separated by a sheet or wall which extends inside the wrapping.

4. Pharmaceutical dosage form according to claim **3**, in which the wrapping and optionally the separating sheet or wall are made of material that is water- soluble at the pH of the mouth, at the pH of the stomach or at the pH of the intestine.

5. Pharmaceutical dosage form according to any one of claims **1** to **4**, comprising two, three or four medicaments.

6. Pharmaceutical dosage form according to any one of claims **1** to **5**, in which the total weight of medicament is less than or equal to 1500 mg, preferably less than or equal to 1000 mg, especially less than or equal to 500 mg.

7. Pharmaceutical dosage form according to any one of the preceding claims, in which the combined medicaments are to be absorbed in the same or a different cavity of the digestive system.

8. Oral pharmaceutical dosage form according to any one of the preceding claims, in which at least one of the medicaments is selected from the following therapeutic classes: non-steroidal anti-inflammatory drug (NSAID), proton pump inhibitor (PPI), beta-blocker, statin, conversion enzyme inhibitor (CEI), biguanide, myorelaxant, calcium inhibitor, corticoid, antidepressant, benzodiazepine, non-atropine-like intestinal transit retarder, intestinal antibacterial, and the following therapeutic molecules: spironolactone, propranolol, clarithromycin, amoxycillin, low-dose acetylsalicylic acid, potassium, clopidogrel.

9. Oral pharmaceutical dosage form according to claim **8** containing medicaments selected to constitute the following combinations:
- a non-steroidal anti-inflammatory drug (NSAID) combined with a proton pump inhibitor (PPI);
- a beta-blocker combined with a statin;
- a conversion enzyme inhibitor (CEI) combined with a statin;
- a statin combined with a biguanide;
- an NSAID combined with an analgesic and/or a myorelaxant; - a calcium inhibitor combined with a CEI;
- spironolactone combined with propranolol;
- a PPI combined with clarithromycin and amoxycillin;
- low-dose acetylsalicyclic acid combined with a CEI, a statin and a beta-blocker;
- low-dose acetylsalicylic acid combined with clopidogrel;
- a corticoid combined with potassium;
- an antidepressant combined with a benzodiazepine;
- a non-atropine-like intestinal transit retarder combined with an intestinal antibacterial.

10. Pharmaceutical dosage form according to claim **8** or **9** containing
- a medicament comprising an NSAID and a medicament comprising a PPI, for the anti-inflammatory treatment of patients at risk of gastroduodenal lesions; or
- a medicament comprising a beta-blocker and a medicament comprising a statin, for the treatment of hypercholesterolaemia in patients with arterial hypertension; or
- a medicament comprising a CEI and a medicament comprising a statin, for the prevention of cardiovascular disorders in at-risk subjects, especially in the case of arterial hypertension associated with hypercholesterolaemia; or
- a medicament comprising a biguanide and a medicament comprising a statin, for the prevention of cardiovascular risks in patients with type 2 diabetes; or
- a medicament comprising an NSAID and a medicament comprising a myorelaxant, for the treatment of common acute lumbar pain; or
- a medicament comprising a calcium inhibitor and a medicament comprising a CEI, for the treatment of arterial hypertension resistant to monotherapy; or
- a medicament comprising spironolactone and a medicament comprising propranolol, for the treatment of cirrhosis in patients with pulmonary arterial hypertension; or
- a medicament comprising a PPI, a medicament comprising clarithromycin and a medicament comprising amoxycillin, for the eradication treatment of the bacterium Helicobacter pylori in the case of gastroduodenal ulcer disease in adults, especially following endoscopic proof of lesion and infection; or
- a medicament comprising low-dose acetylsalicylic acid, a medicament comprising a CEI, a medicament comprising a statin and a medicament comprising a beta-blocker, for post-infarct treatment; or
- a medicament comprising low-dose acetylsalicylic acid and a medicament comprising clopidogrel, for the prevention of the risk of thromboembolism in hypertensive patients and in patients who have undergone angioplasty; or
- a medicament comprising a corticoid and a medicament comprising potassium, for the prevention of hypokaliaemia in patients requiring treatment by corticotherapy; or
- a medicament comprising an antidepressant, a medicament comprising a benzodiazepine, for the treatment of depression; or
- a medicament comprising a non-atropine-like intestinal transit retarder and a medicament comprising an intestinal antibacterial, for the reestablishment of intestinal transit and the treatment of bacterial diarrhoea.

11. Pharmaceutical dosage form according to claim **10**, in which
- the biguanide is selected from: metformin, gliclazide, carbutamide and glibenclamide; or
- the myorelaxant is thiocolchicoside; or
- the calcium inhibitor is selectedfrom: nifedipine and nicardipine; or
- potassium is a potassium salt; or
- the corticoid is selected from: prednisolone and prednisone; or
- the non- atropine-like intestinal transit retarder is loperamide; or
- the antibacterial is nifuroxazide ; or
- the NSAID is selected from: naproxen, ketoprofen, diclofenac and ibuprofen; or
- the CEI is selected from ramipril, captopril, enalapril and fosinopril; or
- the PPI is selected from: omeprazole, esomeprazole and lansoprazole; or
- the statin is selected from: pravastatin, simvastatin; or
- the beta-blocker is selected from: bisoprolol, atenolol, acebutolol, carvedilol; or
- the antidepressant is a tricyclic antidepressant, for example clomipramine, or a selective serotonin reuptake inhibitor, for example fluoxetine or venlafaxine; or
- the benzodiazepine is selected from bromazepan and alprazolam.

## Patentansprüche

1. Orale pharmazeutische Dosierungsform, die mindestens zwei Medikamente enthält, wobei die Medikamente in dieser Form unter der Form von getrennt vorkonstituierten Tabletten sind, die einerseits in einer auslaufsicheren, wasserlöslichen *in vivo* Umhüllung zusammengebracht werden, und andererseits getrennt werden, sodass die Wirkstoffe in den kombinierten Medikamenten nicht in Kontakt miteinander kommen können, und wobei die Umhüllung aus zwei Blättern eines pharmazeutischen Materials und mindestens einem Trennblatt gefertigt ist, das aus pharmazeutischem Material gefertigt ist.

2. Pharmazeutische Dosierungsform nach Anspruch **1**, wobei die Medikamente handelsübliche Tabletten und/oder Tabletten sind, die dieselbe qualitative und quantitative Zusammensetzung wie handelsübliche Tabletten, jedoch eine unterschiedliche geometrische Form aufweisen.

3. Pharmazeutische Dosierungsform nach Anspruch **1** oder **2**, wobei die Medikamente durch ein Blatt oder eine Wand getrennt sind, das/die sich innerhalb der Umhüllung erstreckt.

4. Pharmazeutische Dosierungsform nach Anspruch **3**, wobei die Umhüllung und optional das Trennblatt oder die Trennwand aus einem Material gefertigt sind, das bei pH-Wert des Mundes, bei pH-Wert des Magens oder bei pH-Wert des Darms wasserlöslich ist.

5. Pharmazeutische Dosierungsform nach einem der Ansprüche **1** bis **4**, zwei, drei oder vier Medikamente umfassend.

6. Pharmazeutische Dosierungsform nach einem der Ansprüche **1** bis **5**, wobei das Gesamtgewicht des Medikaments kleiner oder gleich 1 500 mg, vorzugsweise kleiner oder gleich 1 000 mg, speziell kleiner oder gleich 500 mg ist.

7. Pharmazeutische Dosierungsform nach einem der vorstehenden Ansprüche, wobei die kombinierten Medikamente im selben oder einem unterschiedlichen Hohlraum des Verdauungssystems zu absorbieren sind.

8. Orale pharmazeutische Dosierungsform nach einem der vorstehenden Ansprüche, wobei mindestens eines der Medikamente aus den folgenden therapeutischen Klassen ausgewählt ist: nichtsteroidaler Entzündungshemmer (NSAID), Protonenpumpenhemmer (PPI), Betablocker, Statin, Converting-Enzymhemmer (CEI), Biguanid, Muskelrelaxans, Kalziuminhibitor, Kortikoid, Antidepressivum, Benzodiazepin, nicht atropinartige Peristaltikhemmer, Darm-Antibiotikum, und den folgenden therapeutischen Molekülen: Spironolakton, Propanolol, Clarithomycin, Amoxicillin, niedrig dosierte Acetylsalicylsäure, Kalium, Clopidogrel.

9. Orale pharmazeutische Dosierungsform nach Anspruch **8**, Medikamente enthaltend, die ausgewählt werden, um die folgenden Kombinationen zu bilden:
- einen nichtsteroidaler Entzündungshemmer (NSAID), kombiniert mit einem Protonenpumpenhemmer (PPI);
- einen Betablocker kombiniert mit Statin;
- einen Converting-Enzymhemmer (CEI) kombiniert mit Statin;
- Statin kombiniert mit Biguanid;
- NSAID, kombiniert mit einem Analgetikum und/ oder einem Muskelrelaxans;
- einen Kalziuminhibitor kombiniert mit einem CEI;
- Spironolakton kombiniert mit Propanolol;
- ein PPI kombiniert mit Clarithromycin und Amoxicillin;
- niedrig dosierte Acetylsalicylsäure kombiniert mit einem CEI, Statin und einem Betablocker;
- niedrig dosierte Acetylsalicylsäure kombiniert mit Clopidogrel;
- ein Kortikoid kombiniert mit Kalium;
- ein Antidepressivum kombiniert mit Benzodiazepin;
- einen nicht atropinartigen Peristaltikhemmer kombiniert mit einem Darm-Antibiotikum.

10. Pharmazeutische Dosierungsform nach Anspruch **8** oder **9**, die Folgendes enthält
- ein Medikament, das ein NSAID und ein Medikament umfasst, das einen PPI für die Entzündungshemmungsbehandlung von Patienten mit einem Risiko von gastroduodenalen Läsionen umfasst; oder
- ein Medikament, das einen Betablocker und ein Medikament umfasst, das Statin zur Behandlung von Hypercholesterinämie bei Patienten mit arterieller Hypertonie umfasst; oder
- ein Medikament, das einen CEI und ein Medikament umfasst, das Statin zur Vorbeugung von kardiovaskulären Erkrankungen bei Risikopatienten, speziell im Falle einer arteriellen Hypertonie in Verbindung mit Hypercholesterinämie umfasst; oder
- ein Medikament, das Biguanid und ein Medikament umfasst, das Statin zur Vorbeugung von kardiovaskulären Risiken bei Patienten mit Typ 2 Diabetes umfasst, oder
- ein Medikament, das einen NSAID und ein Medikament umfasst, das ein Muskelrelaxans zur Behandlung von allgemeinen akuten lumbalen Schmerzen umfasst; oder
- ein Medikament, das einen Kalziuminhibitor und ein Medikament umfasst, das einen CEI zur Behandlung von arterieller Hypertonie umfasst, das der Monotherapie widersteht; oder
- ein Medikament, das Spironolacton und ein Medikament umfasst, das Propanolol zur Behandlung von Zirrhose bei Patienten mit pulmonaler arterieller Hypertonie umfasst; oder
- ein Medikament, das einen PPI, ein Medikament, das Clarithromycin umfasst, und ein Medikament, das Amoxicillin zur Eradikationsbehandlung des Bakteriums Helicobacter pylori im Falle einer gastroduodenalen Geschwürerkrankung bei Erwachsenen, speziell infolge eines endoskopischen Beweises einer Läsion und Infektion umfasst; oder
- ein Medikament, das eine niedrig dosierte Acetylsalicylsäure umfasst, ein Medikament, das einen CEI umfasst, ein Medikament, das Statin und ein Medikament umfasst, das einen Betablocker zur Behandlung nach einem Infarkt umfasst; oder
- ein Medikament, das eine niedrig dosierte Acetylsalicylsäure und ein Medikament umfasst, das Clopidogrel zur Vorbeugung des Risikos einer Thromboembolie bei hypertonischen Patienten und bei Patienten umfasst, die eine durchgeführte Angioplastie aufweisen; oder
- ein Medikament, das ein Kortikoid und ein Medikament umfasst, das Kalium zur Vorbeugung von Hyperkaliämie bei Patienten umfasst, die eine Behandlung durch Koritkoidtherapie benötigen; oder
- ein Medikament, das ein Antidepressivum, ein Medikament umfasst, das Benzodiazepin zur Behandlung einer Depression umfasst; oder
- ein Medikament, das einen nicht atropinartigen Peristaltikhemmer und ein Medikament umfasst, das ein Darm-Antibiotikum zur Wiederherstellung der Darmtätigkeit und der Behandlung von bakteriellem Durchfall umfasst.

11. Pharmazeutische Dosierungsform nach Anspruch **10**, wobei
- das Biguanid ausgewählt ist aus: Metformin, Gliclazid, Carbutamid und Glibenclamid; oder
- das Muskelrelaxans Thiocolchicosid ist; oder
- der Kalziuminhibitor ausgewählt ist aus: Nifedipin und Nicardipin; oder
- Kalium ein Kaliumsalz ist; oder
- das Kortikoid ausgewählt ist aus: Prednisolon und Prednison; oder
- der nicht atropinartige Peristaltikhemmer Loperamid ist; oder
- das Antibiotikum Nifuroxazid ist; oder
- NSAID ausgewählt ist aus: Naproxen, Ketoprofen, Diclofenac und Ibuprofen; oder
- CEI ausgewählt ist aus: Ramipril, Captopril, Enalapril und Fosinopril; oder
- PPI ausgewählt ist aus: Omeprazol, Esomeprazol und Lansoprazol; oder
- Statin ausgewählt ist aus: Pravastatin, Simvastatin; oder
- der Betablocker ausgewählt ist aus: Bisoprolol, Atenolol, Acebutolol, Carvedilol; oder
- das Antidepressivum ein trizyklisches Antidepressivum, beispielsweise Clomipramin, oder ein selektiver Serotonin-Wiederaufnahmehemmer, beispielsweise Fluotexin oder Venlafaxin ist; oder
- das Benzodiazepin ausgewählt ist aus Bromazepan und Alprazolam.

## Revendications

1. Forme de dosage pharmaceutique orale contenant au moins deux médicaments, dans laquelle les médicaments se présentent sous la forme de comprimés préconstitués séparément qui, d'une part, sont réunis dans un emballage hydrosoluble *in vivo* étanche et, d'autre part, sont séparés de telle sorte que les principes actifs des médicaments combinés ne puissent pas entrer en contact l'un avec l'autre, et dans laquelle l'emballage est constitué de deux feuilles de matériau pharmaceutique, et d'au moins une feuille de séparation en matériau pharmaceutique.

2. Forme de dosage pharmaceutique selon la revendication **1**, dans laquelle les médicaments sont des comprimés commerciaux et/ou des comprimés ayant la même composition qualitative et quantitative que les comprimés commerciaux mais une forme géométrique différente.

3. Forme de dosage pharmaceutique selon la revendication **1** ou **2**, dans laquelle les médicaments sont séparés par une feuille ou une paroi qui s'étend à l'intérieur de l'emballage.

4. Forme de dosage pharmaceutique selon la revendication **3**, dans laquelle l'emballage et optionnellement la feuille ou la paroi de séparation sont constitués d'un matériau soluble dans l'eau au pH de la bouche, au pH de l'estomac ou au pH de l'intestin.

5. Forme de dosage pharmaceutique selon l'une quelconque des revendications **1** à **4**, comprenant un, deux, ou trois médicaments.

6. Forme de dosage pharmaceutique selon l'une quelconque des revendications **1** à **5**, dans laquelle le poids total du médicament est inférieur ou égal à 1500 mg, de préférence inférieur ou égal à 1000 mg, en particulier inférieur ou égal à 500 mg.

7. Forme de dosage pharmaceutique selon l'une quelconque des revendications précédentes, dans laquelle les médicaments combinés doivent être absorbés dans la même cavité ou dans une cavité différente du système digestif.

8. Forme de dosage pharmaceutique selon l'une quelconque des revendications précédentes, dans laquelle au moins un des médicaments est choisi parmi les classes thérapeutiques suivantes : anti-inflammatoire non stéroïdien (AINS), inhibiteur de la pompe à protons (IPP), bêta-bloquant, statine, inhibiteur de l'enzyme de conversion (IEC), biguanide, myorelaxant, inhibiteur du calcium, corticoïde, antidépresseur, benzodiazépine, retardateur du transit intestinal non atropinique, antibactérien intestinal, et les molécules thérapeutiques suivantes : spironolactone, propranolol, clarithromycine, amoxycilline, acide acétylsalicylique à faible dose, potassium, clopidogrel.

9. Forme de dosage pharmaceutique orale selon la revendication **8**, contenant des médicaments sélectionnés pour constituer les combinaisons suivantes :
- un anti-inflammatoire non stéroïdien (AINS) combiné à un inhibiteur de la pompe à protons (IPP) ;
- un bêta-bloquant combiné à une statine ;
- un inhibiteur de l'enzyme de conversion (IEC) combiné à une statine ;
- une statine combinée à un biguanide ;
- un AINS combiné à un analgésique et/ou un myorelaxant ;
- un inhibiteur du calcium combiné à un IEC ;
- une spironolactone combinée à un propranolol ;
- un IPP associé à la clarithromycine et à l'amoxycilline ;
- de l'acide acétylsalicylique à faible dose associé à un IEC, une statine et un bêta-bloquant ;
- de l'acide acétylsalicylique à faible dose combiné à du clopidogrel ;
- un corticoïde combiné à du potassium ;
- un antidépresseur associé à une benzodiazépine ;
- un retardateur du transit intestinal non analogue à l'atropine combiné à un antibactérien intestinal.

10. Forme de dosage pharmaceutique selon la revendication **8** ou **9**, contenant
- un médicament comprenant un AINS et un médicament comprenant un IPP, pour le traitement anti-inflammatoire des patients à risque de lésions gastroduodénales ; ou
- un médicament comprenant un bêta-bloquant et un médicament comprenant une statine, pour le traitement de l'hypercholestérolémie chez les patients souffrant d'hypertension artérielle ; ou
- un médicament comprenant une IEC et un médicament comprenant une statine, pour la prévention des troubles cardiovasculaires chez les sujets à risque, notamment en cas d'hypertension artérielle associée à une hypercholestérolémie ; ou
- un médicament comprenant un biguanide et un médicament comprenant une statine, pour la prévention des risques cardiovasculaires chez les patients atteints de diabète de type 2 ; ou
- un médicament comprenant un AINS et un médicament comprenant un myorelaxant, pour le traitement des douleurs lombaires aiguës courantes ; ou
- un médicament comprenant un inhibiteur du calcium et un médicament comprenant un IEC, pour le traitement de l'hypertension artérielle résistant à la monothérapie ; ou
- un médicament comprenant de la spironolactone et un médicament comprenant du propranolol, pour le traitement de la cirrhose chez les patients souffrant d'hypertension artérielle pulmonaire ; ou
- un médicament comprenant un IPP, un médicament comprenant de la clarithromycine et un médicament comprenant de l'amoxycilline, pour le traitement d'éradication de la bactérie Helicobacter pylori dans le cas d'un ulcère gastroduodénal chez l'adulte, en particulier à la suite de la preuve endoscopique d'une lésion et d'une infection ; ou
- un médicament comprenant de l'acide acétylsalicylique à faible dose, un médicament comprenant une CEI, un médicament comprenant une statine et un médicament comprenant un bêta-bloquant, pour le traitement post-infarctus ; ou
- un médicament comprenant de l'acide acétylsalicylique à faible dose et un médicament comprenant du clopidogrel, pour la prévention du risque de thromboembolie chez les patients hypertendus et chez les patients ayant subi une angioplastie ; ou
- un médicament comprenant un corticoïde et un médicament comprenant du potassium, pour la prévention de l'hypokaliémie chez les patients nécessitant un traitement par corticothérapie ; ou
- un médicament comprenant un antidépresseur, un médicament comprenant une benzodiazépine, pour le traitement de la dépression ; ou
- un médicament comprenant un retardateur de transit intestinal non analogue à l'atropine et un médicament comprenant un antibactérien intestinal, pour le rétablissement du transit intestinal et le traitement de la diarrhée bactérienne.

11. Forme de dosage pharmaceutique selon la revendication **10**, dans laquelle
- le biguanide est choisi parmi metformine, gliclazide, carbutamide et glibenclamide ; ou
- le myorelaxant est le thiocolchicoside ; ou
- l'inhibiteur de calcium est choisi parmi : la nifédipine et la nicardipine ; ou
- le potassium est un sel de potassium ; ou
- le corticoïde est choisi parmi : la prednisolone et la prednisone ; ou
- le retardateur de transit intestinal non atropinique est le lopéramide ; ou
- l'antibactérien est le nifuroxazide ; ou
- l'AINS est choisi parmi : naproxène, kétoprofène, diclofénac et ibuprofène ; ou
- l'IEC est choisi parmi le ramipril, le captopril, l'énalapril et le fosinopril ; ou
- le PPI est choisi parmi : l'oméprazole, l'esoméprazole et le lansoprazole ; ou
- la statine est choisie parmi : pravastatine, simvastatine ; ou
- le bêta-bloquant est choisi parmi : bisoprolol, aténolol, acébutolol, carvédilol ; ou
- l'antidépresseur est un antidépresseur tricyclique, par exemple la clomipramine, ou un inhibiteur sélectif du recaptage de la sérotonine, par exemple la fluoxétine ou la venlafaxine ; ou
- la benzodiazépine est sélectionnée parmi le bromazépan et l'alprazolam.
